# EUROPÄISCHE PATENTSCHRIFT

(11) **EP 2 468 227 B1**
(45) Veröffentlichungstag und Bekanntmachung des Hinweises auf die Patenterteilung: **17.07.2013**
(21) Anmeldenummer: 10016029.0
(22) Anmeldetag: 23.12.2010
(51) Int. Cl.: A61F 13/02, A61F 13/15, A61F 5/40

(54) **Neues kinesiologisches Tape**
New kinesio tape
Nouvelle bande kinésiologique

(43) Veröffentlichungstag der Anmeldung: 27.06.2012
(73) Patentinhaber: Wüst, Dirk, 79650 Schopfheim (DE)
(72) Erfinder: Wüst, Dirk, 79650 Schopfheim (DE)
(74) Vertreter: Schöllhorn, Andreas

(56) Entgegenhaltungen:
- WO-A1-88/03397
- DE-A1- 4 240 952
- DE-A1- 4 431 918
- DE-A1-102005 017 587
- DE-A1-102006 026 395
- US-A- 3 618 754
- US-A- 5 861 348
- US-A1- 2010 298 747

## Beschreibung

Die vorliegende Erfindung betrifft ein dermales druckempfindliches und elastisches Klebeband (Tape), insbesondere ein neues klneslologlsches Tape.

Bereits 1870 entwickelte der japanische Chiropraktiker Kenzo Käse das Kineslo Tape, ein hochelastisches, atmungsaktives, wasserresistentes und hautfreundliches Pflaster. Wie eine zweite Haut dehnt sich das Tape und zieht sich wieder zusammen, ohne die Bewegungsfreiheit einzuschränken. Dabei wirkt es Schmerz-dämpfend und Stoffwechsel-steigernd und unterstützt die Bewegung.

Das Kinesio-Tape besteht zu 100% aus Baumwolle. Das Baumwollgewebe ist mit einer Acrylbesohlohtung versehen. Durch die Acrylbeschlehtung Ist das Tape selbstklebend. Die luft- und wasserdurchlässige Gewebestruktur des Baumwoll-Tapes hat etwa die Elastizität und das Gewicht der Haut und Ist In alle Richtungen dehnbar. Der Begriff "Kineslo-Tape" ist in Fachkrelsen bekannt. Das Tape ist im Handel erhältlich z.B. bel Biviax GmbH, Dortmund.

Die Elastizität von 130 bis 140% eines Kinesio-Tapes ist vergleichbar mit der Elgendehnung des menschlichen Muskels. Diese Elastizität in Verbindung mit einer speziellen Klebetechnik ermöglicht die Normalisierung der Muskelfunktion und die Förderung der Lymph- und Blutzirkulation.

Die Acrylbeschichtung des Klnesio-Tapes hat eine wellenförmige Struktur. Die Beschichtung ist sinusförmig angeordnet mit einer unbeschichteten Lücke zwischen den Beschichtungen. Dadurch ist das Tape Luft- und Feuchtigkelts-durchlässig. Die Klebeeigenschaft der Beschichtung wird über die Körperwärme aktiviert.

US5861348 (Kinesio. Corp. 1999) beschreibt ein Kinesio-Tape. In Fg.1 ist die wellenförmige Struktur der Acrylbeschichtung erkennbar.

DE 4240952 beschreibt einen elastichen Verband mit Noppen zur Einwirkung auf Akupressurpunkte.

Trotz der erfolgreichen Anwendung des Kinesio-Tapes in verschiedenen medizinischen Fachgebieten, besteht ein Bedarf das klassische Kinesio Tape weiterzuentwickeln, um die Wirkung zu optimieren und somit noch effektiver zu machen.

Es wurde nun gefunden, dass eine deutliche Steigerung der Effektivität durch eine Kombination aus einem Kinesio-Tape und neuen Faszilatoren gegeben ist.

Die Erfindung betrifft somit ein dermales Klebeband bestehend aus einem dehnbaren Textilmaterial und einer Klebeschicht, wobei sich auf der Klebeschicht eine Vielzahl von Faszilatoren befindet. (Anspruch 1)

Das dehnbare Textilmaterial ist in allen Richtungen elastisch.
Die Dehnbarkeit beträgt 120-150%, beispielsweise 130-140%.

Das Textilmaterial besteht vorzugsweise aus Baumwolle, (Anspruch 2)
Es sind aber auch andere Materialien denkbar, die bel Verbänden zum Einsatz kommen wie z.B. Vliesstoffe, Cellulose, Polyester, Polyamid, Acetat und Mischgewebe.

Das Textlimaterial kann gefärbt sein, sodass das erfindungsgemässe Klebeband in allen Farben vorliegen kann.

Die Klebeschicht darf die Haut nicht reizen und besteht vorzugsweise aus einer Acrylschicht. (Anspruch 3)
Es sind aber auch andere Klebstoffe denkbar, die bei Verbänden zum Einsatz kommen, wie z.B. Kautschukkleber.
Die Klebesohicht bedeckt das Textilmaterial. Vorzugsweise ist das Textilmaterial nicht vollständig bedeckt, sondern es gibt unbeschichtete Bahnen oder Streifen, die zwischen den Beschichtungen verlaufen in verschiedenen Formen z.B. wellenförmig, streifenförmig, etc.

Zum Schutz der Klebschicht trägt das Klebeband in der Verkaufsform eine Schutzschicht vorzugsweise aus Papier.

In einer bevorzugten Ausführungsform Ist das Klebeband (Schicht 1 und 2) ein Kinesio-Tape. (Anspruch 4).
Beim Kinesio-Tape ist das Textilmaterial aus Baumwolle. Die Klebeschicht ist eine Acrylklebeschicht, die wellenförmig verläuft. Zwischen den Acrylkiebeschichtwellen befindet sich unbeschichtete Baumwolle. Die unbeschichtete Baumwolle verläuft ebenfalls wellenförmig.

Die Faszilatoren sind in einer Ausführungsform kleine Partikel.
Die Faszilatorpartikel können Jede beliebige Form haben wie z.B. Kugel, Ellipsoid, Polyeder (z.B. Tetraeder) Würfel, Pyramide, Torus, Kegel, Prisma usw. Die Partikel auf der

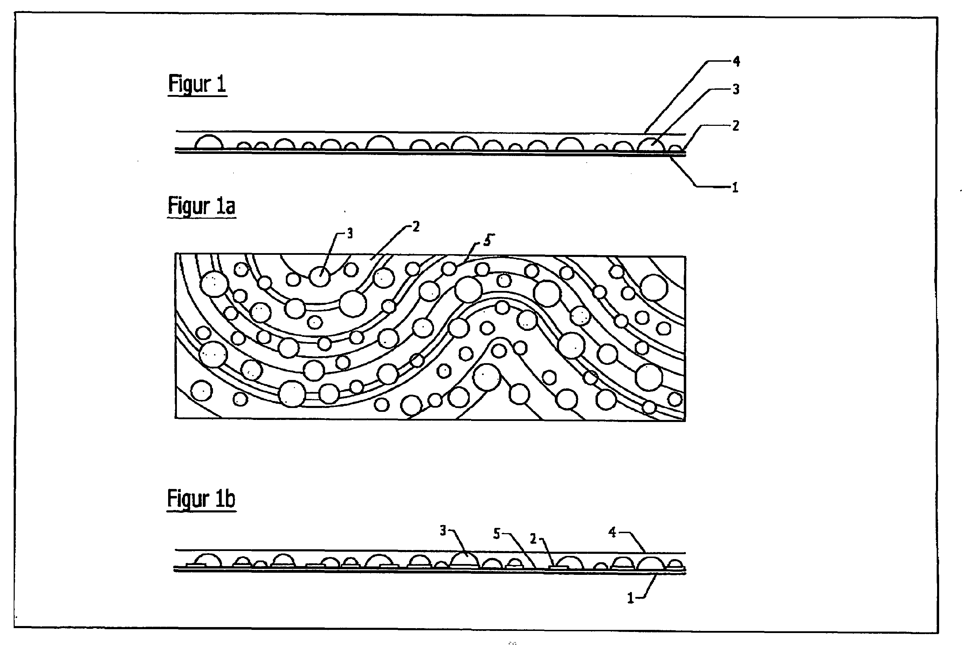

Klebeschicht können alle dieselbe Form haben oder verschiedene Formen. In Fig.1 sind kugelförmige Faszilatorpartikel dargestellt.

Länge, Breite und Höhe der Faszilatorpartikel ist variabel.
Beispielsweise beträgt die Höhe 0.1-20 mm oder 0.1-15 mm, vorzugsweise 0.5-5 mm. Länge und Breite liegen beispielsweise zwischen 1-10 mm. Diese Angaben sind lediglich Richtwerte. Die Faszilatorpartikel können auch grösser sein.

Die Verteilung der Faszilatorpartikel auf der Klebeschicht ist entweder ungleichmässig (zufällig) oder gleichmässig. Eine gleichmässige Anordnung der Faszilatorpartikel wäre beispielsweise eine Anordnung der Partikel In Reihen.

Vorteilhafterweise ist die gesamte Klebeschicht mit Faszilatorpartikeln versehen. (Anspruch 5).
Die Anzahl der Fasziliatorpartikel Ist variable und beträgt z.B. 50 - 200 Partikel /100cm². Es ist aber auch möglich, dass nur ein Tell der Kiebeschicht mit Faszilatorpartikeln bedeckt ist

In einer weiteren Ausführungsform sind die Faszilatoren Bänder.
Die Faszilatorbänder können netzartig oder In Reihen auf der Klebeschicht aufgebracht werden. Die Faszilatorbänder sind in Länge, Breite und Höhe ebenfalls variabel. Beispielsweise beträgt die Höhe 0.1-20 mm oder 0.1-15 mm, vorzugsweise 0.5-5 mm.

Die Faszilatoren bestehen aus jedem möglichen Material was hautverträglich Ist, sich zu Partikeln oder Bändern formen lässt und sich auf die Kiebeschicht aufkleben lässt.

Die Faszilatoren sind beispielsweise aus hautverträglichem Kunststoff. (Anspruch 6) Hautverträgliche Kunststoffe sind beispielsweise Styropor, Gummi, thermoplastische Kunststoffe, Silikone, Polyolefine, usw.
Beispielsweise sind die Faszilatorpartikel aus Styropor. (Anspruch 7)
Die Faszilatoren können aber auch aus natürlichen Materialien wie Holz, Sand oder Baumwolle sein.

Die Faszilatoren dürfen nicht zu hart sein, sodass keine Druckstellen entstehen.

Wichtig ist eine gute Anhaftung der Faszilatoren an der Kiebeschicht 2.

Die Faszilatoren (Partikel oder Bänder) können auch selbst auf ihrer Oberfläche eine Klebeschicht tragen. Durch diese zusätzliche Klebeschicht lässt sich die Anhaftung an der Haut nach Auflegen des Tapes noch steigern.

Die Faszilatoren (Partikel oder Bänder) können in verschiedenen Farben vorliegen.
Auch die Klebeschicht kann gefärbt sein.

In einer Ausführungsform weist das erfindungsgemässe Klebeband einen basischen p_{H}-Wert auf. (Anspruch 8)
Dies wird erreicht durch Eintauchen des Klebebands in eine basische Lösung wie z.B. in eine NaHCO₃ Lösung und anschliessende Trocknung oder durch Besprühen des Klebebands mit der basischen Lösung. Das basische Klebeband hat zusätzlich den Vorteil, dass das Bindegewebe stärker über die Haut entsäuert werden kann. Der p_{H}-Wert liegt beispielsweise zwischen 8-10.

Werden als Faszilatoren benetzbare Materialien verwendet, wie z.B. Methacrylat-Copolymere oder natürliche Materialien so ist es auch möglich die Faszilatoren mit der basischen Lösung zu besprühen oder sie in die basische Lösung einzutauchen.

In der basischen Lösung können auch Mineralien gelöst werden, sodass ein Klebaband hergestellt werden kann, welches Mineralien beinhaltet (Anspruch 9).
Die gelösten Mineralien fördern die Ausscheidung von Giftstoffen und Schlacken über die Haut. Mineralien sind beispielsweise solche, die in käuflichen basischen Mineralpulvern verwendet werden, wie z.B. Kalium-, Calcium-. Magnesium- Salze usw.

Das erfindungsgemässe Klebeband kommt zur Anwendung als orthopädische Bandage. (Anspruch 12)

Das erfindungsgemässe Klebeband dient zur Herstellung eines Tape-Verbandes zur Anwendung In therapeutischen Verfahren, vorzugsweise zur Behandlung von Gelenkerkrankunpen oder Sportverietzungen. (Anspruch 10 und 11).

Beschreibung der Figuren:
Fig. 1 zeigt einen schematischen Aufbau der erfindungsgemässen Kombination. Auf einem dehnbaren Textilmaterial 1 befindet sich die Klebeschicht 2, worauf sich die Faszilatoren 3 befinden. Die Faszilatoren 3 kleben fest an der Klebschicht 2 an, sodass sie beim Anlegen des Tapes nicht verrutschen und sich nicht lösen können. Zum Schutz der Klebschicht trägt das Klebeband in der Verkaufsform eine Schutzschicht 4 vorzugsweise aus Papier.
Fig. 1 a zeigt eine Draufsicht auf das erfindungsgemässe Klebeband. Die runden Faszilatorpartikel 3 sind auf der gesamten Oberfläche der Klebeschicht 2 verteilt. ist das Klebeband ein Kinesio-Tape, ist die Klebeschicht 2 wellenförmig angeordnet. Das unbeschichtete Textilmaterial 5 verläuft ebenfalls wellenförmig. Die Faszilatorpartikel befinden sich entweder vollständig im Bereich der Klebeschicht oder ragen in das unbeschichtete Textilmaterial 5 hinein.
Fig. 1b zeigt einen Querschnitt des erfindungsgemässen Klebebands. Das Textilmaterial 1 ist mit der Klebeschicht 2 beschichtet. Es gibt auch unbeschichtete Bereiche 5 des Textilmaterials. Auf der Klebeschicht befinden sich die Faszilatorpartikel 3, darüber die Schutzschicht 4.

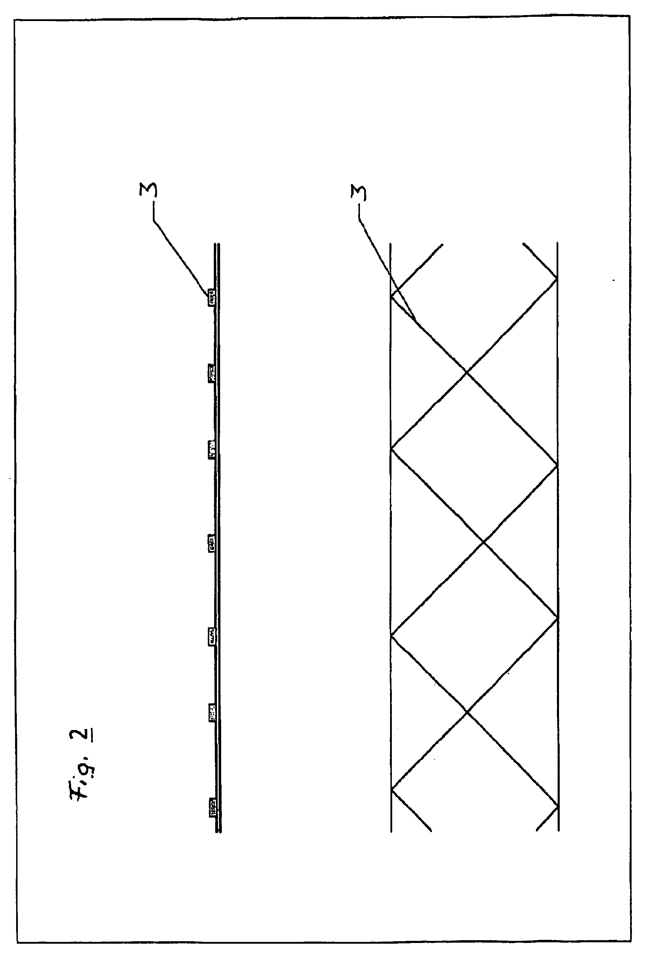

Fig. 2 zeigt einen Querschnitt und eine Draufsicht auf das erfindungsgemässe Klebeband. Die Faszilatoren 3 sind Bänder, die netzartig angeordnet sind.

Das Kinesio-Tape wirkt u. a. durch den Hautfalteneffekt.
Wird die Haut im betroffenen Bereich durch die Tapeanlage gedehnt, bildet die Haut zusammen mit dem aufgeklebten Kinosio-Tape bei der Rückführung in den Ruhezustand wellenförmige Hautfälten aus. Durch das Anheben der Haut vergrößert sich der Raum zwischen Haut und Unterhautgewebe. Dadurch kann die Lymphflüssigkeit aus den Zwischenräumen einfacher in das Lymphsystem zurückfliesen und die Reizung der Hautrezeptoren wird vermindert sowie die Seibstheilungseffekte des Körpers unterstützt.

### Vorteil des erfindungsgemässen Klebebands:

Die Faszilatoren üben zusätzlich einen leichten Druck auf die Haut aus. Bei Bewegung der Haut bewirken die Faszilatoren einen verstärkten Effekt der Mikromassage des Unterhautbindegewebes, wodurch die folgenden Bereiche positiv unterstützt werden.
Hautsensoren und Schmerzrezeptoren
Propriozeptoren
Muskulatur und Muskelansätze
Fasziales Gewebe
Bänder und Kapselstrukturen
Blut - und Lymphsystem
Meridiane und Akupunkturpunkte
Narbengewebe
Cranlo-Sacrales System
Viezerales System.

Das erfindungsgemässe Klebeband wirkt durch die Faszilatoren spannungslösend, schmerzlindernd und Stoffwechseisteigernd.

Die richtige Handhabung des Tapes ist Grundvoraussetzung für die optimale Wirkung und Haftung des erfindungsgemässen Tapes.

Je nach Situation unterscheidet man verschiedene Techniken.
1. Die zu behandeinde Hautstelle wird in eine Vordehnung gebracht. Das Tape selber wird ohne Zug aufgeklebt.
2. Wenn man die zu behandeinde Hautstelle nicht in eine Vordehnung bringen kann, sollte das Tape mit einem leichten Zug auf beiden Seiten von ca. 3cm ungedehnten Taperand angebracht werden.
3. Bei Schmerzen die genau lokalisiert werden können, sollte das Tape grundsätzlich gedehnt aufgebracht werden. Wichtig dabei wieder auf beiden Seiten einen von ca. 3cm ungedehnten Taperand lassen.
4. Die Enden werden mit einer Schere abgerundet, weil das Tape sich auf diese Art besser haftet.
5. Das Tape sollte ca. 30-60 min vor einem Wettkampf aufgeklebt werden.
6. Es ist möglich die Tape-enden mit Pflasterspray oder Chloraethylspray zu besprühen und ca. 3 min trocknen zu lassen.

## Patentansprüche

1. Dermales Klebeband bestehend aus einem dehnbaren Textilmaterial und einer Klebeschicht, wobei sich auf dieser Klebeschicht eine Vielzahl von Faszilatoren befindet, wobei die Faszilatoren Partikel oder bandförmig sind.

2. Dermales Klebeband gemäss Anspruch 1, wobei das Textilmaterial Baumwolle ist.

3. Dermales Klebeband gemäss Anspruch 1, wobei die Klebeschicht eine Acrylatschicht ist.

4. Dermales Klebeband gemäss einem der Ansprüche 1-3, wobei das Klebeband ein Kinesio-Tape ist.

5. Dermales Klebeband gemäss einem der Ansprüche 1-4, wobei die Faszilatorpartikel auf der gesamten Klebeschicht verteilt sind.

6. Dermales Klebeband gemäss einem der Ansprüche 1-5, wobei die Faszilatoren aus einem hautverträglichen Kunststoff bestehen.

7. Dermales Klebeband gemäss Anspruch 6, wobei die Faszilatoren aus Styropor sind.

8. Dermales Klebeband gemäss einem der Ansprüche 1-7, wobei das Klebeband einen basischen pH-Wert aufweist.

9. Dermales Klebeband nach Anspruch 8, wobei das Klebeband Mineralien beinhaltet.

10. Dermales Klebeband wie in Anspruch 1-9 definiert zur Herstellung eines Tape-Verbandes zur Anwendung in therapeutischen Verfahren.

11. Dermales Klebeband wie in Anspruch 1-9 definiert zur Herstellung eines Tape-Verbandes zur Behandlung von Gelenkerkrankungen oder Sportverletzungen.

## Claims

1. Dermal adhesive tape consisting of a elastic textile material and an adhesive layer, wherein a plurality of fascial bodies is arranged on this adhesive layer, wherein the fascial bodies are particles or ribbon-shaped.

2. Dermal adhesive tape according to claim 1, wherein the textile material is cotton.

3. Dermal adhesive tape according to claim 1, wherein the adhesive layer is an acrylate layer.

4. Dermal adhesive tape according to any one of claims 1-3, wherein the tape is a Kinesiology tape.

5. Dermal adhesive tape according to any one of claims 1-4, wherein the fascial particles are distributed over the entire adhesive layer.

6. Dermal adhesive tape according to any one of claims 1-5, wherein the fascial bodies consist of a skin-compatible plastic.

7. Dermal adhesive tape according to claim 6, wherein the fascial bodies are made of polystyrene.

8. Dermal adhesive tape according to any one of claims 1-7, wherein the tape has an alkaline pH value.

9. Dermal adhesive tape according to claim 8, wherein the tape includes minerals.

10. Dermal adhesive tape as defined in claim 1-9 for manufacturing a tape bandage for use in therapeutic applications.

11. Dermal adhesive tape as defined in claim 1-9 for manufacturing a tape bandage for treating joint diseases or sports injuries.

## Revendications

1. Ruban adhésif cutané comportant un matériau textile extensible et une couche adhésive, une pluralité des fascialateurs se trouvant sur cette couche adhésive, les fascialateurs étant des particules ou en la forme de ruban.

2. Ruban adhésif cutané selon la revendication 1, le matériau textile étant coton.

3. Ruban adhésif cutané selon la revendication 1, la couche adhésive étant une couche d'acrylate.

4. Ruban adhésif cutané selon l'une des revendications 1 à 3, le ruban adhésif étant une bande kinésiologique.

5. Ruban adhésif cutané selon l'une des revendications 1 à 4, les particules des fascialateurs étant répartis sur l'intégralité de la surface adhésive.

6. Ruban adhésif cutané selon l'une des revendications 1 à 5, les fascialateurs consistant en un matériau plastique possédant une bonne tolérance cutanée.

7. Ruban adhésif cutané selon la revendication 6, les fascialateurs étant en polystyrène.

8. Ruban adhésif cutané selon l'une des revendications 1 à 7, le ruban adhésif possédant une valeur de pH basique.

9. Ruban adhésif cutané selon la revendication 8, le ruban adhésif contenant des minéraux.

10. Ruban adhésif cutané comme défini á la revendication 1 à 9 pour la fabrication d'un bandage adhésif à utilisation dans des procédures thérapeutiques.

11. Ruban adhésif cutané comme défini á la revendication 1 à 9 pour la fabrication d'un bandage adhésif pour le traitement de l'arthropathie ou de blessures sportives.
